(19) Europäisches Patentamt
European Patent Office
Office européen des brevets

(11) EP 4 467 129 A1

(12) # EUROPEAN PATENT APPLICATION
published in accordance with Art. 153(4) EPC

(43) Date of publication:
**27.11.2024 Bulletin 2024/48**

(21) Application number: **23780717.7**

(22) Date of filing: **29.03.2023**

(51) International Patent Classification (IPC):
***A61K 9/127*** *(2006.01)* ***A61K 31/47*** *(2006.01)*
***A61K 47/02*** *(2006.01)* ***A61K 47/12*** *(2006.01)*
***A61K 47/14*** *(2017.01)* ***A61K 47/24*** *(2006.01)*
***A61K 47/26*** *(2006.01)* ***A61K 47/28*** *(2006.01)*
***A61P 35/00*** *(2006.01)* ***C07D 215/48*** *(2006.01)*

(52) Cooperative Patent Classification (CPC):
**A61K 9/127; A61K 31/47; A61K 47/02; A61K 47/12; A61K 47/14; A61K 47/24; A61K 47/26; A61K 47/28; A61P 35/00; C07D 215/48**

(86) International application number:
**PCT/JP2023/012862**

(87) International publication number:
**WO 2023/190709 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.03.2022 JP 2022060823**

(71) Applicant: **Eisai R&D Management Co., Ltd.**
**Tokyo 112-8088 (JP)**

(72) Inventors:
- **MATSUMOTO Yasunobu**
**Tsukuba-shi, Ibaraki 300-2635 (JP)**
- **HYODO Kenji**
**Tsukuba-shi, Ibaraki 300-2635 (JP)**
- **TAKASE Yasutaka**
**Tsukuba-shi, Ibaraki 300-2635 (JP)**
- **KATSURADA Yuri**
**Tsukuba-shi, Ibaraki 300-2635 (JP)**
- **ANDO Yuichiro**
**Tsukuba-shi, Ibaraki 300-2635 (JP)**
- **ONIZAWA Yuji**
**Tsukuba-shi, Ibaraki 300-2635 (JP)**
- **SUZUKI Takuya**
**Kakamigahara-shi, Gifu 501-6024 (JP)**

(74) Representative: **Hoffmann Eitle**
**Patent- und Rechtsanwälte PartmbB**
**Arabellastraße 30**
**81925 München (DE)**

(54) **LIPOSOME COMPOSITION AND LIPOSOME-CONTAINING PHARMACEUTICAL COMPOSITION**

(57) A liposome composition comprising (A) liposomes comprising a dihydrosphingomyelin and a diacylglycerol-polyethylene glycol, (B) a drug, and (C) a sulfuric acid salt and/or sucrose octasulfate, and in which (A) encapsulates (B), wherein the diacylglycerol-polyethylene glycol is distearoylglycerol-polyethylene glycol, and (B) is 4-(3-chloro-4-(ethylaminocarbonyl)aminophenoxy)-7-methoxy-6-quinolinecarboxamide represented by formula (I) or its pharmaceutically acceptable salt.

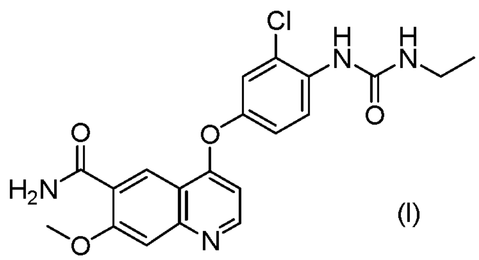
(I)



Processed by Luminess, 75001 PARIS (FR)

(Cont. next page)

# Fig.1

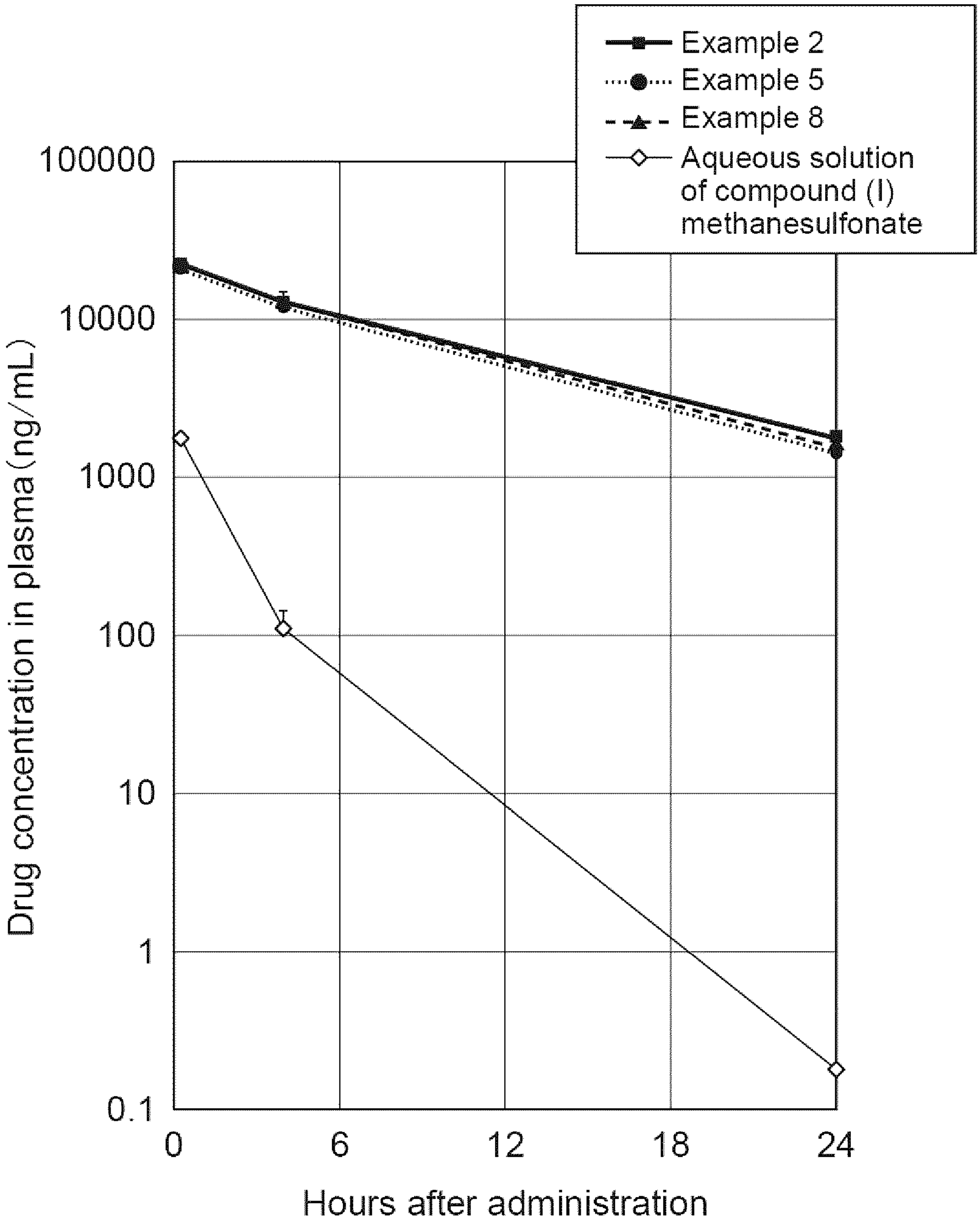
Example 2
Example 5
Example 8
Aqueous solution of compound (I) methanesulfonate
100000
10000
1000
100
10
1
0.1
Drug concentration in plasma (ng/mL)
0
6
12
18
24
Hours after administration

# Description

## Technical Field

The present invention relates to a liposome composition and to a liposome-containing pharmaceutical composition.

## Background Art

Liposomes are microscopic closed vesicles comprising an inner layer surrounded by one or more lipid bilayers, and they are generally able to hold a water-soluble substance inside the inner phase while holding a fat-soluble substance on the lipid bilayer. Research is being conducted on formulations using liposomal techniques, whereby this property of liposomes is used to encapsulate drugs in liposomes for delivery to target tissue. Examples of hitherto proposed formulations using liposomal techniques include liposome compositions containing diacylphosphatidylethanolamine, a dihydrosphingomyelin and cholesterols, modified with a hydrophilic polymer (PTL 1).

Receptor tyrosine kinase inhibitors are known as molecular targeted agents exhibiting antitumor activity via inhibition of tyrosine kinase on cell membranes and inside cells. Lenvatinib (chemical name: 4-(3-chloro-4-(cyclopropylaminocarbonyl)aminophenoxy)-7-methoxy-6-quinolinecarboxamide) is a tyrosine kinase inhibitor targeting receptors such as vascular endothelial growth factor receptors (VEGFR)1-3 and fibroblast growth factor receptors (FGFR)1-4 (PTL 2), and PTL 3 discloses formulations using liposomal technology which contain lenvatinib as a drug. PTL 1 also discloses a liposome composition containing sunitinib (a tyrosine kinase inhibitor) as a drug.

## Citation List

### Patent Literature

[PTL 1] International Patent Publication No. WO2018/181963
[PTL 2] US Patent No. 7253286
[PTL 3] Chinese Patent Application Publication No. 112603890

## Summary of Invention

### Technical Problem

It is an object of the invention to provide a novel liposome composition with excellent retentivity in the blood, containing a tyrosine kinase inhibitor as a drug.

### Solution to Problem

The present inventors have completed this invention after finding, unexpectedly, that a liposome composition containing liposomes composed of a specific lipid and comprising 4-(3-chloro-4-(ethylaminocarbonyl)aminophenoxy)-7-methoxy-6-quinolinecarboxamide represented by the following formula (I) (hereunder also referred to as "compound (I)") or its pharmaceutically acceptable salt as a drug with tyrosine kinase inhibiting activity, has high retentivity in the blood.

Specifically, the disclosure provides the following [1] to [19].

[1] A liposome composition comprising:

(A) liposomes comprising a dihydrosphingomyelin and a diacylglycerol-polyethylene glycol,
(B) a drug, and
(C) a sulfuric acid salt and/or sucrose octasulfate,

wherein

(A) encapsulates (B),
the diacylglycerol-polyethylene glycol is distearoylglycerol-polyethylene glycol, and
(B) is 4-(3-chloro-4-(ethylaminocarbonyl)aminophenoxy)-7-methoxy-6-quinolinecarboxamide represented by

formula (I) or its pharmaceutically acceptable salt.

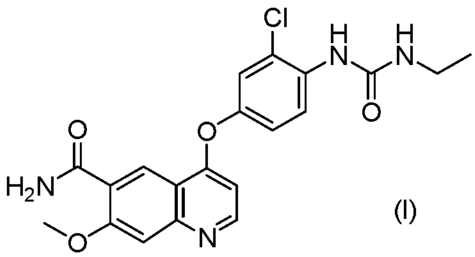

(I)

[2] The liposome composition according to [1], wherein (C) is a sulfuric acid salt.
[3] The liposome composition according to [1] or [2], wherein the dihydrosphingomyelin is a compound represented by formula (II-1), (II-2), (II-3) or (II-4), or their mixture.

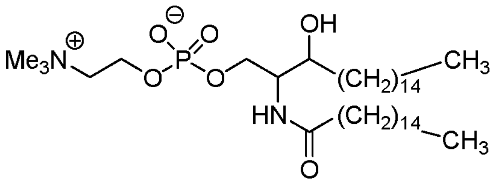

(II-1)

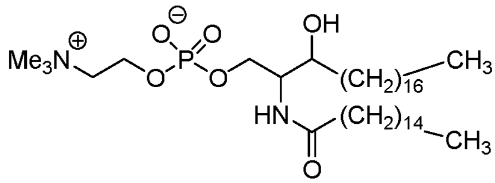

(II-2)

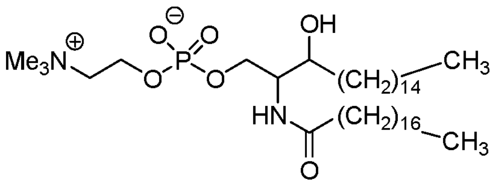

(II-3)

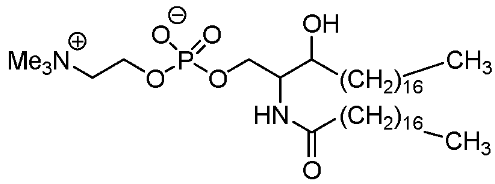

(II-4)

[4] The liposome composition according to [1] or [2], wherein the dihydrosphingomyelin is a compound represented by formula (II-3).

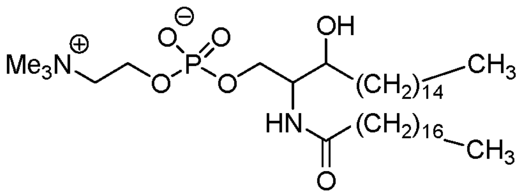

(II-3)

[5] The liposome composition according to any one of [1] to [4], further comprising
(D) an organic acid.
[6] The liposome composition according to [5], wherein (D) is one or more selected from the group consisting of succinic acid, citric acid, maleic acid and salicylic acid.
[7] The liposome composition according to any one of [1] to [6], wherein (A) further comprises a cholesterol.
[8] The liposome composition according to any one of [1] to [7], further comprising
(E) a basic compound.
[9] The liposome composition according to any one of [1] to [8], wherein (C) is ammonium sulfate.
[10] The liposome composition according to any one of [5] to [9], wherein (D) is succinic acid.
[11] The liposome composition according to any one of [8] to [10], wherein (E) is sodium hydroxide or ammonia.
[12] The liposome composition according to any one of [1] to [11], wherein (A) further encapsulates (C).
[13] A pharmaceutical composition comprising a liposome composition according to any one of [1] to [12].
[14] An angiogenesis inhibitor comprising a liposome composition according to any one of [1] to [12].
[15] An antitumor agent comprising a liposome composition according to any one of [1] to [12].
[16] A method for prevention or treatment of a tumor in a subject, the method comprising administration to the subject of a liposome composition according to any one of [1] to [12].
[17] Use of the liposome composition according to any one of [1] to [12] for production of a drug for prevention or treatment of a tumor.

[18] A liposome composition according to any one of [1] to [12], to be used for prevention or treatment of a tumor.
[19] A method for producing a liposome composition according to any one of [1] to [12], the method comprising:

a step of providing a liposome dispersion, and
a step of mixing 4-(3-chloro-4-(ethylaminocarbonyl)aminophenoxy)-7-methoxy-6-quinolinecarboxamide represented by formula (I) or its pharmaceutically acceptable salt with the liposome dispersion.

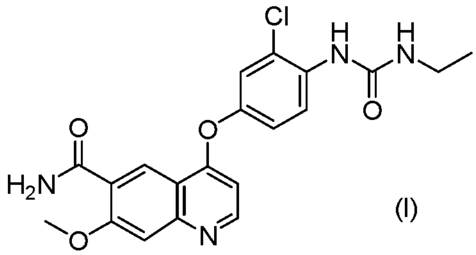

(I)

## Advantageous Effects of Invention

According to the invention it is possible to provide a novel liposome composition with excellent retentivity in the blood, containing a tyrosine kinase inhibitor as a drug.

## Brief Description of Drawings

Fig. 1 is a graph showing change in drug blood concentrations, with intravenous administration to mice of the liposome compositions of Examples 2, 5 and 8, and an aqueous solution of a methanesulfonic acid salt of compound (I). Fig. 2 is a graph showing the results of an evaluation test for anti-PEG IgM antibody production.

## Description of Embodiments

As used herein, "liposome" means a microscopic closed vesicle having an inner phase encapsulated by a lipid bilayer. The term "liposome" encompasses small unilamellar vesicles (SUV), large unilamellar vesicles (LUV), giant unilamellar vesicles (GUV), multilamellar vesicles (MLV) as liposomes having multiple concentric membranes, and multivesicular vesicles (MVV) as liposomes having multiple irregular membranes.
As used herein, "liposome inner phase" refers to the region of the liposome encapsulated by the lipid bilayer, and when the region contains water, it is synonymous with "inner aqueous phase" and "liposome inner aqueous phase". The term "liposome outer phase" refers to the region of the liposome that is not encapsulated by the lipid bilayer when the liposome is dispersed in a liquid (that is, the region other than the inner phase and lipid bilayer).
The liposome composition of this embodiment comprises (A) liposomes comprising a dihydrosphingomyelin and diacylglycerol-polyethylene glycol (hereunder also referred to as "component (A)"), (B) a drug (hereunder also referred to as "component (B)"), and (C) a sulfuric acid salt and/or sucrose octasulfate (hereunder also referred to as "component (C)").
The liposome composition of the embodiment encapsulates component (B) as a drug in the inner phase of the liposomes. The term "encapsulates", as used herein, means a form in which the actual membranes of the liposomes encapsulate the drug, a form in which the drug is encapsulated within the closed spaces formed by the liposome membranes, or any combination of these forms.

<u>(A) Liposomes</u>

The liposome composition of the embodiment comprises (A) liposomes that comprise a dihydrosphingomyelin and diacylglycerol-polyethylene glycol.
A dihydrosphingomyelin is a phospholipid having two carbon chains in the molecule, and it is useful as a lipid for further increasing blood retentivity of the drug-encapsulating liposome composition used for the invention. More specifically, it may be a compound represented by formula (II-1), (II-2), (II-3) or (II-4), or their mixture.

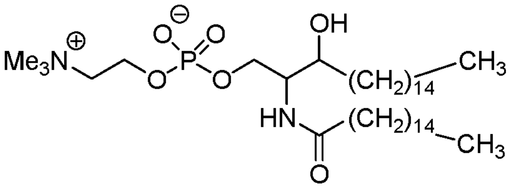 (II-1)

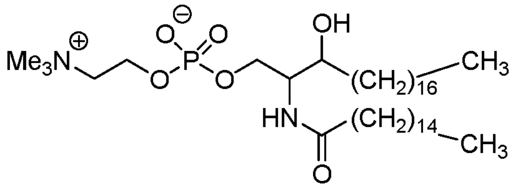 (II-2)

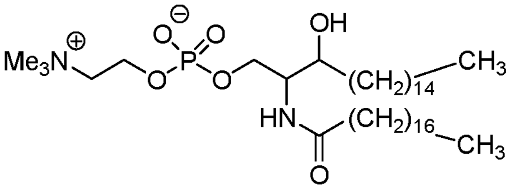 (II-3)

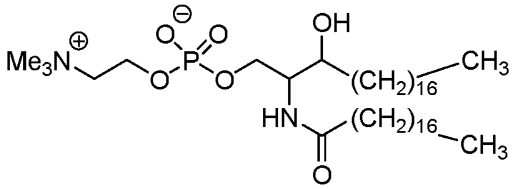 (II-4)

The dihydrosphingomyelin used may be, for example, a dihydrosphingomyelin obtained by reducing a naturally derived sphingomyelin by a common method, or a dihydrosphingomyelin obtained by chemical synthesis (synthetic dihydrosphingomyelin). A synthetic dihydrosphingomyelin preferably has a long carbon chain and high purity, so as to exhibit superior retentivity in the blood. Dihydrosphingomyelins derived naturally, such as from hen eggs, are generally compounds represented by formula (II-1) above whereas chemical synthesis can produce compounds represented by formulas (II-1) to (II-4) at high purity.

The content of the dihydrosphingomyelin in the total lipid component of the liposomes is not particularly restricted and may be 40 to 99.9 mol%, 45 to 96 mol% or 48 to 64 mol%.

A diacylglycerol-polyethylene glycol is a compound wherein the hydroxyl group of a diacylglycerol is modified with polyethylene glycol. The molecular weight of the polyethylene glycol in the diacylglycerol-polyethylene glycol is not particularly restricted but is preferably 500 to 10,000 daltons, and more preferably 1000 to 5000 daltons. Examples of diacylglycerol-polyethylene glycols include dimyristoylglycerol-polyethylene glycol (DMG-PEG), dipalmitoylglycerol-polyethylene glycol (DPG-PEG) and distearoylglycerol-polyethylene glycol (DSG-PEG). One embodiment of a diacylglycerol-polyethylene glycol is distearoylglycerol-polyethylene glycol (for example, DSG-PEG2000).

The content of the diacylglycerol-polyethylene glycol in the total lipid component of the liposomes is not particularly restricted and may be 0.1 to 15 mol%, 0.2 to 10 mol% or 0.3 to 6 mol%.

For this embodiment, component (A) may further include a cholesterol. This can further increase the membrane stability of the liposomes.

Examples of cholesterols include cholesterol, 3-cholestanone and acylcholesterol.

When component (A) contains a cholesterol for this embodiment, the content of the cholesterol in the total lipid component of the liposomes is not particularly restricted and may be 0.1 to 50 mol%, 10 to 50 mol% or 30 to 48 mol%.

When component (A) contains a cholesterol for this embodiment, the content ratio of the cholesterol with respect to the dihydrosphingomyelin in component (A) is not particularly restricted, but it may be 15 to 60 parts by mass, 20 to 50 parts by mass or 21 to 35 parts by mass, from the viewpoint of increasing the encapsulation rate of the drug (component (B)).

The content of component (A) in the liposome composition is not particularly restricted, but it is preferably 0.001 to 50 mass%, more preferably 0.01 to 40 mass% and even more preferably 0.01 to 30 mass%, based on the total mass of the liposome composition.

The mean particle size of the liposomes is not particularly restricted, but it may be 20 to 500 nm, 50 to 300 nm or 70 to 200 nm, from the viewpoint of reproducibility of production and degree of accumulation at the tumor site. The mean particle size of the liposomes can be measured by the dynamic light scattering method.

<u>(B) Drug</u>

The liposome composition of the embodiment comprises (B) compound (I) or its pharmaceutically acceptable salt as a drug.

Compound (I) (CAS No. 417719-46-1) is a publicly known compound.

Compound (I) in the liposome composition of the embodiment may be in free form or in the form of a pharmaceutically acceptable salt. Examples of pharmaceutically acceptable salts include salts of inorganic acids, salts of organic acids, salts of inorganic bases, salts of organic bases, and salts of acidic or basic amino acids.

Preferred examples of inorganic acid salts include salts of hydrochloric acid, hydrobromic acid, sulfuric acid, nitric acid and phosphoric acid. Examples of salts of organic acids include salts of acetic acid, succinic acid, fumaric acid, maleic acid, tartaric acid, citric acid, lactic acid, stearic acid, benzoic acid, methanesulfonic acid (mesylic acid), ethanesulfonic

acid andp-toluenesulfonic acid.

Examples of salts of inorganic bases include alkali metal salts such as sodium salts and potassium salts, alkaline earth metal salts such as calcium salts and magnesium salts, and aluminum salts and ammonium salts. Examples of salts of organic bases include diethylamine, diethanolamine, meglumine and N,N-dibenzylethylenediamine.

Examples of salts of acidic amino acids include salts of aspartic acid and glutamic acid. Examples of salts of basic amino acids include salts of arginine, lysine and ornithine.

Compound (I) or its pharmaceutically acceptable salt to be used for preparation of the liposome composition of the embodiment can be produced by the method described in Example 583 of PTL 1, for example.

A pharmaceutically acceptable salt of compound (I) used for preparation of the liposome composition of the embodiment is not particularly restricted so long as it is one of the aforementioned salts, and it may be a salt of an organic acid such as maleic acid, for example.

The content of component (B) in the liposome composition of the embodiment is not particularly restricted, and may be 0.001 to 100 mg/mL, 0.05 to 80 mg/mL, 0.01 to 50 mg/mL or 0.1 to 30 mg/mL. Likewise, the content of component (B) in the liposome composition of the embodiment is not particularly restricted but may be 0.01 to 50 mass%, 0.1 to 40 mass%, 0.5 to 30 mass% or 1 to 20 mass%, based on the total mass of the liposome composition.

The drug/total lipid weight ratio (D/L ratio) in the liposome composition of the embodiment may be greater than 0.1, for example. The D/L ratio in the liposome composition of the embodiment may also be 1.0 or lower, for example. The D/L ratio can be determined by calculating the total lipid concentration from the cholesterol concentration of the liposome composition based on the following formula:

$$\text{Total lipid concentration (mg/mL)} = \text{Cholesterol concentration (mg/mL)} \times \frac{\text{Weighed total lipid weight (mg)}}{\text{Weighed cholesterol weight (mg)}}$$

and dividing the drug concentration of the liposome composition by the calculated total lipid concentration. If the D/L ratio is a high value of greater than 0.1 it will be possible to administer a smaller lipid amount during administration of the necessary amount of drug, thereby helping to further reduce load on the body such as lipid-induced toxicity.

(C) Sulfuric acid salt and/or sucrose octasulfate

The liposome composition of the invention comprises (C) a sulfuric acid salt and/or sucrose octasulfate. If the inner aqueous phase of the liposome includes component (C), it will be possible to increase the encapsulation rate and blood retentivity of the drug (component (B)).

The content of component (C) in the inner aqueous phase of the liposomes is not particularly restricted, and may be 10 to 1000 mM, for example.

The sulfuric acid salt may be ammonium sulfate, hydrogen ammonium sulfate or triethylamine sulfate, for example.

The content of the sulfuric acid salt in the inner aqueous phase of the liposomes is not particularly restricted, and may be 50 to 1000 mM, 100 to 500 mM or 150 to 400 mM, for example.

Sucrose octasulfate may be sucrose octasulfate-triethylamine (SOS-TEA) or ammonium sucrose octasulfate, for example.

The content of the sucrose octasulfate in the inner aqueous phase of the liposomes is not particularly restricted, and may be 10 to 200 mM, 20 to 150 mM or 30 to 100 mM, for example.

One embodiment of component (C) is a sulfuric acid salt. Another embodiment of component (C) is ammonium sulfate.

(D) Organic acid

The liposome composition of the embodiment may further comprise (D) an organic acid (hereunder also referred to as "component (D)"). If the inner aqueous phase of the liposomes further includes component (D), this may be expected to provide an effect of inhibiting decomposition of the constituent components of the liposome. Examples of organic acids include those with buffer capacity, such as succinic acid, citric acid, maleic acid and salicylic acid. One embodiment of the organic acid is succinic acid.

The content of component (D) in the inner phase of the liposomes is not particularly restricted, and may be 0.1 to 300 mM, 5 to 200 mM or 10 to 100 mM, for example.

(E) Basic compound

The liposome composition of the embodiment may further comprise a basic compound (hereunder also referred

to as "component (E)"). Examples of basic compounds include alkali metal hydroxides (such as sodium hydroxide and potassium hydroxide), and amines (such as ammonia and alkylamines). One embodiment of the basic compound is sodium hydroxide. Another embodiment of the basic compound is ammonia.

Inner aqueous phase pH

The pH of the inner aqueous phase in the liposome composition of the embodiment is not particularly restricted and may be 1 to 6, for example.

Method for producing liposome composition

The method for producing the liposome composition of the embodiment is not particularly restricted, and for example, it can be produced by steps of preparing a lipid mixture, preparing an aqueous phase, formation of liposome particles, particle size adjustment, replacement of the outer aqueous phase of the liposomes, encapsulation of a drug into the liposome particles, and removal of the outer aqueous phase drug.

<Preparation of lipid mixture>

For preparation of the lipid mixture, each component of the liposomes (the dihydrosphingomyelin, the diacylglycerol-polyethylene glycol, and the cholesterol as necessary) may be mixed with an organic solvent, and the mixture may be heated to dissolve the components, thereby producing an oil phase. The organic solvent used for the oil phase is not particularly restricted, and for example, a water-soluble organic solvent that is optionally miscible with water may be used. Alternatively, an organic solvent which is not water-soluble may be used for preparation, mixing and dissolving the components and then removing the organic solvent.

Examples of water-soluble organic solvents include alcohols such as methanol, ethanol, n-propanol, isopropanol, n-butanol, isobutanol and t-butanol, glycols such as glycerin, ethylene glycol and propylene glycol, and polyalkylene glycols such as polyethylene glycol. Alcohols are preferred among these. Alcohols are preferably one or more selected from among ethanol, methanol, 2-propanol and t-butanol, more preferably one or more selected from among ethanol, 2-propanol and t-butanol, and even more preferably ethanol.

<Preparation of aqueous phase>

The aqueous phase used may be water (such as distilled water or water for injection), physiological saline, a buffering solution or an aqueous solution of a saccharide (such as sucrose), or a mixture (aqueous solvent) of the same. According to the invention, when the drug is to be encapsulated into the liposome particles by the remote loading method described below, an aqueous solution comprising component (C) may be used as the aqueous phase.

The buffering solution is not limited to either an organic system or inorganic system, and it is preferably a buffering solution having buffering action close to the approximate hydrogen ion concentration of body fluids, examples of which include phosphate buffer, Tris buffer, citrate buffer, acetate buffer, histidine buffering solution or Good's buffer. The inner phase of the liposomes may be an aqueous solution that disperses the liposomes during production of the liposomes, or it may be newly added water, physiological saline, a buffering solution or an aqueous solution of a saccharide, or their mixture. The water to be used in the outer aqueous phase or inner aqueous phase may be one that does not include impurities (such as dirt or chemical substances).

Physiological saline is an inorganic salt solution prepared so as to be isotonic with the human body, and it may also have a buffering function. The physiological saline may be brine comprising sodium chloride at 0.9 w/v% (weight/volume percent), PBS, or Tris-buffered saline.

As used herein, the term "aqueous phase" encompasses both the outer aqueous phase and inner aqueous phase. The term "outer aqueous phase" as used herein means an aqueous solution that disperses the liposomes. In the case of an injection, for example, the outer aqueous phase is the solution occupying the outer sides of the liposomes in the liposome dispersion that has been packaged and stored in a vial or prefilled syringe. Likewise, for a prepared solution for dispersion, or a solution to be dispersed at the time of administration using another solution, the outer aqueous phase is the solution occupying the outer sides of the liposomes of the liposome dispersion. The term "inner aqueous phase", as used herein, means the aqueous phase inside the closed vesicles separated by the lipid bilayer membranes of the liposomes.

<Formation of liposome particles>

The liposomes are formed by mixing the lipid mixture with the aqueous phase. The liquid temperature for formation of the liposome particles may be appropriately adjusted.

<Particle size adjustment>

The obtained liposomes may have their particle sizes uniformly adjusted by extrusion treatment. Extrusion treatment means applying physical shear force by passing the liposomes through a filter with pores, for atomization.

<Replacement of liposome outer aqueous phase liquid>

For this embodiment, when the drug is to be encapsulated into liposome particles by the remote loading method described below, the liposome outer aqueous phase liquid may be replaced. The replacement liquid may be, but is not limited to, a 0.05 to 5 mass% sodium chloride aqueous solution, a 0.05 to 20 mass% glucose aqueous solution or a 0.05 to 40 mass% sucrose aqueous solution. The replacement liquid can be used to remove the aqueous solution comprising component (C) which is present in the outer aqueous phase, by dialysis, ultracentrifugation or ultrafiltration of the liposome solution, to obtain liposomes with the outer aqueous phase replaced by the replacement liquid.

<Encapsulation of drug into liposome particles by remote loading>

According to this embodiment, the drug may be encapsulated into the liposome particles by a remote loading method. The term "remote loading method" as used herein means a method in which liposomes not encapsulating the drug are produced by the method described above, and the drug is added to the outer phase of the liposomes to introduce the drug into the liposomes. Component (C) (such as ammonium sulfate) may be used for remote loading.

In remote loading, the drug added to the outer aqueous phase migrates into the liposomes and becomes incorporated into the liposomes. The driving force for this process may be a solubility gradient, ion gradient or pH gradient. One example is a method of using an ion gradient formed across the liposome membranes, to introduce the drug into the liposome interiors. The temperature during encapsulation of the drug into the liposome particles by remote loading is not particularly restricted, and it may be 20 to 90°C, for example.

<Removal of outer aqueous phase drug and adjustment of drug concentration>

The liposome solution encapsulating the drug may be subjected to dialysis, ultracentrifugation or filtration to remove the drug not included in the liposomes. At the same time, sucrose/histidine buffer at a prescribed concentration may be used as a replacement liquid, with adjustment to the desired drug concentration by ultracentrifugation or ultrafiltration.

<Sterile filtration and aseptic filling>

The obtained liposome composition may be treated by sterile filtration and/or aseptic filling. Sterile filtration can be carried out using a filter with a sterilizable pore size (for example, a 0.2 μm filtration-sterile filter). The method used for aseptic filling may be any publicly known method.

The liposome composition of the embodiment exhibits an effect of excellent retentivity in the blood. Retentivity in the blood can be evaluated (when the liposome composition is administered to an animal such as a mouse, for example), using the drug concentration in the blood after 24 hours as the marker. As demonstrated by the Examples described below, the liposome composition of the embodiment has excellent retentivity in the blood, exhibiting a high value of above 800 ng/mL as the drug concentration in the blood of mice after 24 hours (with a drug dose of 2 mg/kg).

The liposome composition of the embodiment can be used as a pharmaceutical composition. The liposome composition of the embodiment may also be used as an angiogenesis inhibitor, or as an antitumor agent. When the liposome composition of the embodiment is used as an antitumor agent, the types of cancer to which it may be applied include pancreatic cancer, stomach cancer, colorectal cancer, breast cancer, prostate cancer, lung cancer, renal carcinoma, brain cancer, blood cancer, ovarian cancer, head and neck cancer, thyroid cancer, esophageal cancer, hepatic cancer, biliary tract cancer, pancreatic and gastrointestinal tract or neuroendocrine cancer, malignant melanoma, endometrial cancer, sarcoma and urothelial cancer.

The pharmaceutical composition of this embodiment can be produced by a known method, such as the method described in General Rules for Preparations of the Japanese Pharmacopoeia, 17th Edition. The pharmaceutical composition of the embodiment may be appropriately administered to a patient in a manner suitable for the dosage form.

The dosage of the pharmaceutical composition of the embodiment will differ depending on the type of target disease, and the age, gender, body weight and severity of symptoms of the patient, but it will usually be about 0.1 to 300 mg per administration for adults, in terms of the free form of compound (1).

Examples

The present invention will now be explained in greater detail by the following examples. However, various other embodiments of the present invention may be implemented, and the invention is not to be interpreted as being limited to the Examples described herein.

The nuclear magnetic resonance spectra were measured using a JEOL 500 (JMTC-500/54/JJ, ECZ500RS) or a Bruker Ultrashield Plus 600 (BZH102/600/70F, D355/54-6026, PABBO 600S3 BBF-H-D-05 Z SP). The chemical shifts in the proton nuclear magnetic resonance spectra are recorded in δ units (ppm) with respect to tetramethylsilane, and the coupling constants are recorded in Hertz (Hz). The abbreviations for the splitting patterns are the following. s: singlet, d: doublet, t: triplet, dd: double doublet, m: multiplet, brs: broad singlet, brd: broad doublet, brt: broad triplet.

1. Measurement of inhibiting effect on cell proliferation by vascular endothelial growth factor (VEGF) or basic fibroblast growth factor (bFGF) stimulation

The inhibiting effect of compound (I) on cell proliferation by VEGF or bFGF stimulation was evaluated by the following procedure based on HUVEC 2D growth assay.
Normal human umbilical vein endothelial cells (HUVEC) were isolated by a previously reported method (Shinseikagaku Jikken Koza, "Cell Culture Technology", p.197-202). The cells were cultured overnight in a collagen I-coated flask (IWAKI 4143-010), with EGM-2 culture medium (Lonza Inc CC-3162) containing 10% Fetal Bovine Serum (FBS: Sigma 172012) in a 5% $CO_2$ incubator (37°C). On the following day, the culture supernatant was discarded and the medium was exchanged with EBM-2 culture medium containing 2% FBS, prior to further culturing overnight. On the following day, 2% FBS, and a HUVEC cell suspension prepared to $1.5 \times 10^4$ cells/mL with Human Endothelial Serum Free Medium (SFM: Gibco 11111-044) containing penicillin/streptomycin (FujiFilm-Wako Pure Chemical Industries) (hereunder referred to as "2% FBS-SFM") were each added at 100 μL to each well of a 96-well plate (Corning 3904), and culturing was carried out overnight in a 5% $CO_2$ incubator (37°C). On the following day, the test substance diluted with 2% FBS-SFM, and VEGF (R&D Systems 293-VE) or bFGF (Gibco 13256-029) prepared with SFM culture medium containing 2% FBS to a final concentration of 20 ng/mL were each added at 50 μL, and the cells were cultured for 3 days in a 5% $CO_2$ incubator (37°C). A 100 μL portion of each culture supernatant was suctioned from each well and discarded, 100 μL of CellTiter-Glo$^R$ 2.0 Assay (Promega G9243) was added to each well, and the mixture was then incubated for 20 minutes at room temperature. The luminescence in each well was measured using ENVISION™ (Perkin-Elmer). The cell survival rates in the presence of the test substance were determined, with 100% as the value measured with addition of VEGF or bFGF but without addition of the test substance, and 0% as the value measured without addition of the test substance or of VEGF or bFGF. The concentration of test substance necessary to produce 50% inhibition of HUVEC proliferation in the presence of VEGF or bFGF ($IC_{50}$) was calculated based on the cell survival rates.
The $IC_{50}$ values for compound (I) for cell proliferation by VEGF and bFGF stimulation were 1.75 nM and 343 nM, respectively.

2. Preparation of liposome composition

(1) Preparation of lipid mixture

Ethanol was mixed in an equal weight amount to the total lipid weights listed in liposome column of Table 1, and the mixture was dissolved at 80°C to obtain a lipid mixture. In Table 1, DSPC represents distearoylphosphatidylcholine, Egg-SM represents hen egg sphingomyelin, Egg-DHSM represents hen egg dihydrosphingomyelin, TS-DHSM represents synthetic dihydrosphingomyelin, DSG-PEG represents distearoylglycerol-polyethylene glycol and Chol. represents cholesterol. There were used Lipoid PC 18:0/18:0 (product of Lipoid) as DSPC, Lipoid E SM (product of Lipoid) as Egg-SM, hydrogenated Egg-SM starting material as Egg-DHSM and SUNBRIGHT GS-020 (product of NOF Corp.) as DSG-PEG. TS-DHSM can be synthesized by the method described below. The nuclear magnetic resonance spectrum for the synthesized TS-DHSM was as follows.
$^1$H-NMR Spectrum (600 MHz, $CD_3OD$) δ(ppm): 0.92 (6H, t, J=7.1 Hz), 1.17-1.45 (56H, m), 1.53-1.64 (3H, m), 1.67 (1H, brd, J=6.4 Hz), 2.19-2.29 (2H, m), 3.24 (9H, s), 3.59-3.69 (3H, m), 3.86-3.93 (1H, m), 3.93-4.06 (1H, m), 4.10-4.18 (1H, m), 4.23-4.33 (2H, m).

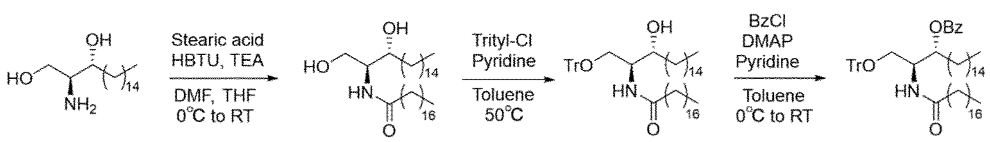

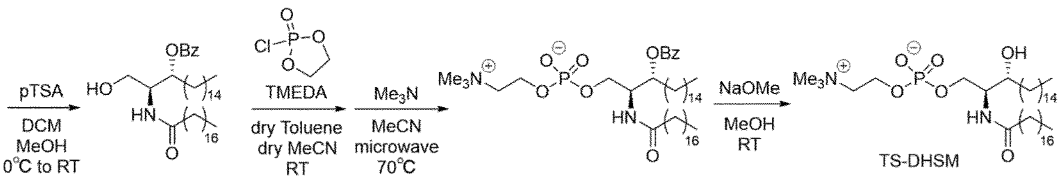


(2) Preparation of aqueous phase

(2a) Preparation of aqueous phase 1 (liposome inner aqueous phase)

In Table 1, each aqueous phase 1 of Examples 1, 2, 10, 11 and 12 and Comparative Examples 1 to 5 was obtained by adding an ammonia water solution to an aqueous solution of sulfuric acid or methanesulfonic acid to adjust the pH to 4, with volume adjustment using water to the concentrations listed in Table 1. The aqueous phase 1 of Example 3 was obtained by mixing the aqueous phase 1 prepared in Examples 1 and 2 and Comparative Examples 1 to 4 and the aqueous phase 1 prepared in Comparative Example 5, in a 1:1 ratio. Examples 4 and 5 were obtained by passing an aqueous solution of sodium sucrose octasulfate (product of Amadis Chemical) through an ion exchange resin for desalting, adjusting the pH to 4 with triethylamine, and then using water to adjust the volume to the concentrations listed in Table 1. Example 6 was obtained by dissolving ammonium sulfate and succinic acid in water, and using water to adjust the volume to the concentration listed in Table 1. Examples 7 to 9 and Comparative Example 6 were obtained by dissolving ammonium sulfate and succinic acid in water and adding an aqueous solution of the bases listed in Table 1 to adjust the pH to 4, and then using water to adjust the volume to the listed concentrations.
As an example, the aqueous phases 1 of Examples 7 and 8 were obtained by weighing out 9.25 g of ammonium sulfate and 1.18 g of succinic acid and adding water (160 mL) for dissolution, adding 5.8 mL of a 1 N sodium hydroxide aqueous solution to adjust the pH to 4, and adjusting the volume to 200 mL with water.

(2b) Preparation of aqueous phase 2 (outer aqueous phase during drug encapsulation)

After dissolving L-histidine (9.3 g) and salt (18 g) in water (1900 mL) and adjusting the pH to 6 with a hydrochloric acid aqueous solution, the volume was adjusted to 2000 mL to obtain aqueous phase 2.

(2c) Preparation of aqueous phase 3 (outer aqueous phase after drug encapsulation)

After dissolving L-histidine (1.55 g) and salt (9 g) in water (900 mL) and adjusting the pH to 7.5 with a hydrochloric acid aqueous solution, the volume was adjusted to 1000 mL to obtain aqueous phase 3.

(3) Preparation of liposomes

Aqueous phase 1 preheated to 80°C was added to a lipid mixture and the components were mixed by inverting. The particle sizes were adjusted by successively passing the mixture through a 100 nm pore size filter (product of Whatman), using an extruder (product of Lipex) while heating at 80°C. The adjusted particles were diluted with physiological saline and then ultracentrifuged at about 300,000 g. The supernatant was removed and the pellet was suspended in physiological saline, and then again ultracentrifuged at about 300,000 g. The supernatant was again removed and the pellet was suspended in physiological saline. The suspension was passed through a PES (polyethersulfone) filter (product of Whatman) having a 0.45 μm pore size, to obtain liposomes.

(4) Preparation of liposome composition (formulation)

The drug (a maleic acid salt of compound (I) or a methanesulfonic acid salt of lenvatinib) was dissolved in free form in a 5% glucose/HCl solution to a concentration of 0.75 mg/mL, to prepare a drug solution. The liposomes and the drug solution were successively added to aqueous phase 2 that had been preheated to 60°C, and mixed for 60 minutes at 60°C. Aqueous phase 2 was prepared so that the mixed drug concentration was 0.2 mg/mL, with the drug solution and liposomes provided for a weight ratio of drug to total lipids of 0.17 to 0.2. After cooling the liquid mixture to 4°C, it was passed through a PES filter (product of Whatman) having a pore size of 0.45 μm. The filtrate was diluted with physiological saline and ultracentrifuged at about 300,000 g, after which the supernatant was removed. The pellet was resuspended in aqueous phase 3 and passed through a PES filter (product of Pall) having a pore size of 0.22 μm, to obtain a liposome composition. The mean particle size of the liposome composition was measured by the dynamic light scattering method using a particle size measuring device (Zetasizer NanoZS (Malvern)).

3. Calculation of drug/total lipid weight ratio (D/L ratio) of liposome composition

The cholesterol concentration and drug concentration of the prepared liposome composition were measured by HPLC under the following conditions.

(3-1) Cholesterol concentration

Detection: UV215 nm
Column: ODS (C18)
Mobile phase: Methanol/tetrahydrofuran/0.17 M ammonium acetate = 94/5/1 (v/v/v)

(3-2) Drug concentration

Detection: UV252 nm
Column: ODS (C18)
Mobile phase A:
Water/acetonitrile/TFA= 990/10/1
Mobile phase B:

Water/acetonitrile/TFA = 100/900/1
Mobile phase B concentration: 20%
The D/L ratio of each liposome composition was calculated by the method described in paragraph [0037] above. The calculation results are shown in Table 1.

4. Measurement of blood drug concentration (1)

The prepared liposome composition was intravenously administered to ICR male mice through the caudal vein (with a dose of 2 mg/kg drug, with 4 mice per group), and at 24 hours after administration, whole blood was collected from the inferior vena cava under isoflurane anesthesia. The blood was centrifuged for 10 minutes at about 1200 g, and the plasma was collected. The drug concentration of the harvested plasma was assayed using LC-MS/MS. The blood drug concentrations at 24 hours after administration are shown in Table 1.

When compound (I) was used as the drug, the blood drug concentration was higher with superior blood retentivity using the liposome compositions of Examples 1 and 2 which contained hen egg dihydrosphingomyelin or synthetic dihydrosphingomyelin in the liposomes, as compared to the liposome compositions of Comparative Examples 1 and 2 which contained distearoylphosphatidylcholine or hen egg sphingomyelin in the liposomes. The liposome compositions of Comparative Examples 3 and 4 which replaced the drug of Examples 1 and 2 from compound (I) with lenvatinib had notably lower blood drug concentrations compared to the liposome compositions of Examples 1 and 2.

[Table 1]

| | Drug | Inner aqueous phase | Lipid (mg) | | | | | | D/L ratio | Blood drug concentration (ng/mL) |
|---|---|---|---|---|---|---|---|---|---|---|
| | | | DSPC | Egg-SM | Egg-DHSM | TS-DHSM | DSG-PEG | Chol. | | |
| Example 1 | Compound (I) | 350 mM ammonium sulfate | - | - | 190.4 | - | 46.5 | 82.2 | 0.14 | 1308 |
| Example 2 | Compound (I) | 350 mM ammonium sulfate | - | - | - | 197.9 | 46.5 | 82.2 | 0.13 | 1752 |
| Example 3 | Compound (I) | 175 mM ammonium sulfate, 175 mM ammonium methanesulfonate | - | - | - | 197.9 | 46.5 | 82.2 | 0.18 | 849 |
| Example 4 | Compound (I) | 80 mM triethylamine sucrose octasulfate | - | - | 190.4 | - | 46.5 | 82.2 | 0.08 | 1220 |
| Example 5 | Compound (I) | 80 mM triethylamine sucrose octasulfate | - | - | - | 198.0 | 46.5 | 82.2 | 0.06 | 1433 |
| Example 6 | Compound (I) | 350 mM ammonium sulfate, 50 mM succinic acid | - | - | - | 197.9 | 46.5 | 82.2 | 0.14 | 1540 |
| Example 7 | Compound (I) | 350 mM ammonium sulfate, 50 mM succinic acid, NaOH | - | - | 190.4 | - | 46.5 | 82.2 | 0.18 | 1180 |
| Example 8 | Compound (I) | 350 mM ammonium sulfate, 50 mM succinic acid, NaOH | - | - | - | 198.0 | 46.4 | 82.2 | 0.14 | 1498 |
| Example 9 | Compound (I) | 350 mM ammonium sulfate, 50 mM succinic acid, ammonia | - | - | - | 197.9 | 46.5 | 82.2 | 0.19 | 1307 |
| Example 10 | Compound (I) | 100 mM ammonium sulfate | - | - | - | 197.9 | 46.5 | 82.2 | 0.03 | 1019 |
| Example 11 | Compound (I) | 250 mM ammonium sulfate | - | - | - | 197.9 | 46.5 | 82.2 | 0.13 | 1438 |
| Example 12 | Compound (I) | 400 mM ammonium sulfate | - | - | - | 197.9 | 46.5 | 82.2 | 0.19 | 1423 |
| Comp. Ex. 1 | Compound (I) | 350 mM ammonium sulfate | 213.4 | - | - | - | 46.5 | 82.2 | 0.07 | 273 |
| Comp. Ex. 2 | Compound (I) | 350 mM ammonium sulfate | - | 189.8 | - | - | 46.5 | 82.2 | 0.10 | 574 |

(continued)

| | Drug | Inner aqueous phase | Lipid (mg) | | | | | | D/L ratio | Blood drug concentration (ng/mL) |
|---|---|---|---|---|---|---|---|---|---|---|
| | | | DSPC | Egg-SM | Egg-DHSM | TS-DHSM | DSG-PEG | Chol. | | |
| Comp. Ex. 3 | Lenvatinib | 350 mM ammonium sulfate | - | - | 190.4 | - | 46.5 | 82.2 | 0.03 | 30 |
| Comp. Ex. 4 | Lenvatinib | 350 mM ammonium sulfate | - | - | - | 197.9 | 46.5 | 82.2 | 0.03 | 47 |
| Comp. Ex. 5 | Compound (I) | 350 mM ammonium methanesulfonate | - | - | - | 197.9 | 46.5 | 82.2 | 0.06 | 328 |
| Comp. Ex. 6 | Lenvatinib | 350 mM ammonium sulfate, 50 mM succinic acid, NaOH | - | - | - | 198.0 | 46.4 | 82.2 | 0.03 | 51 |

5. Measurement of blood drug concentration (2)

The liposome compositions of Examples 2, 5 and 8, and an aqueous solution of methanesulfonic acid salt of compound (I), were administered to mice by the same method as "4. Measurement of blood drug concentration (1)", collecting blood at 15 minutes, 4 hours and 24 hours after administration, and measuring the change in blood drug concentration. The results are shown in Fig. 1.

The liposome compositions of Examples 2, 5 and 8 exhibited satisfactory change in blood drug concentration at 15 minutes, 4 hours and 24 hours after administration, while the aqueous solution of the methanesulfonate of compound (I) had decreased blood drug concentration after administration, being significantly lower after 24 hours. This confirmed that the blood retentivity after administration of the aqueous solution of the methanesulfonic acid salt of compound (I) is notably increased by administration as a liposome composition of the embodiment.

6. Anti-PEG IgM antibody production evaluation test

It is known that repeated administration of polyethylene glycol-modified liposomes to a subject produces the phenomenon of Accelerated Blood Clearance (ABC) whereby anti-PEG antibodies are produced and blood retentivity of a drug from the second administration onward is reduced. By comparing amounts of anti-PEG antibody production thought to be due to the ABC phenomenon, it is possible to evaluate the inhibiting effect on the phenomenon. Liposome compositions having compound (I) encapsulated in liposomes of the present embodiment and liposomes without encapsulation of compound (I) were administered to mice, and the level of anti-PEG antibody production in each of the mice was evaluated by the following method.

A liposome composition encapsulating compound (I) prepared in the same manner as Example 8, and liposomes before encapsulation of compound (I), were intravenously administered to mice through the tail at a dose of 4 mg/kg of DHSM (1 mg/kg as the drug for the liposome composition encapsulating compound (I)), for each BALB/c male mouse. The dosage of DHSM was measured by the same method as for measurement of the cholesterol concentration described above in paragraph [0073], (3-1). Serum was collected from the mice administered the liposome composition encapsulating compound (I) and the mice administered liposomes without encapsulation of compound (I), by sampling whole blood from the inferior vena cava under isoflurane anesthesia at 10 days after administration. The collected mouse sera were diluted 100-fold and, following the method reported in Reference A, the absorbance at 490 nm was measured using a spectrophotometer (SpectraMax M2 (product of Molecular Devices)), to evaluate the anti-PEG IgM antibody production level (Reference A: International Journal of Pharmaceutics 15(2012) 436:636-643). The results are shown in Fig. 2.

Serum harvested from the mice administered the liposome composition encapsulating compound (I) had significantly lower anti-PEG IgM antibody concentration compared to serum harvested from mice administered the liposomes without encapsulation of compound (I). This confirmed that combination of liposomes of the embodiment with compound (I) has an effect of inhibiting the ABC phenomenon. In other words, combining the liposome composition of the embodiment with compound (I) or its pharmaceutically acceptable salt inhibits the ABC phenomenon, so that high retentivity in the blood can be expected from the second administration onward.

Maleic acid salts and methanesulfonic acid salts of compound (I) can be produced by the following exemplary methods. However, these specific examples are merely illustrative and are not intended to restrict the invention in any way.

Commercial products were used as appropriate for the compounds in the Production Examples. The abbreviations used have the following meanings.

$CD_3OD$: Deuterated methanol
DMSO-$d_6$: Deuterated dimethyl sulfoxide
DMA: N,N-Dimethylacetamide

[Production Example 1]

4-(3-Chloro-4-(ethylaminocarbonyl)aminophenoxy)-7-methoxy-6-quinolinecarboxamide maleate

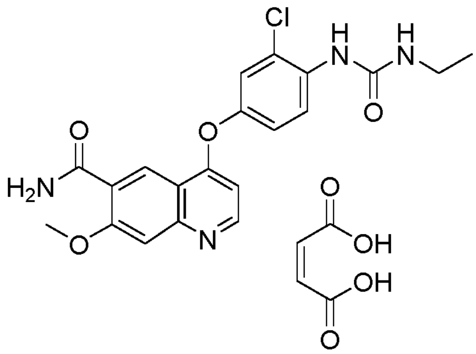

After adding 4-(3-chloro-4-(ethylaminocarbonyl)aminophenoxy)-7-methoxy-6-quinolinecarboxamide (5.70 g, 13.7 mmol) to a DMA solvent (23 mL), the mixture was dissolved by heated stirring at 80°C, and then a solution of maleic acid (1.91 g, 16.5 mmol) in DMA(5.5 mL) was added and stirring was continued for 5 minutes. After adding 2-butanone (350 mL) dropwise to the reaction mixture over a period of 1 hour, it was stirred for 17 hours at 40°C. The reaction mixture was cooled to room temperature and stirred for 6 hours, after which the precipitated solid was filtered. The obtained solid was further washed with 2-butanone and then subjected to ventilation drying and reduced pressure drying at 70°C to obtain the title compound (6.40 g).
$^1$H-NMR Spectrum (500 MHz, DMSO-$d_6$) $\delta$(ppm): 1.08 (3H, t, J=7.2 Hz), 3.10-3.18 (2H, m), 4.04 (3H, s), 6.23 (2H, s), 6.60 (1H, d, J=5.5 Hz), 7.00 (1H, t, J=5.5 Hz), 7.25 (1H, dd, J=9.0, 2.8 Hz), 7.50 (1H, d, J=2.8 Hz), 7.53 (1H, s), 7.76 (1H, brs), 7.87 (1H, brs), 8.08 (1H, s), 8.28(1H, d, J=9.6 Hz), 8.67 (1H, s), 8.72 (1H, d, J=4.8 Hz).

[Production Example 2]

4-(3-Chloro-4-(ethylaminocarbonyl)aminophenoxy)-7-methoxy-6-quinolinecarboxamide methanesulfonate

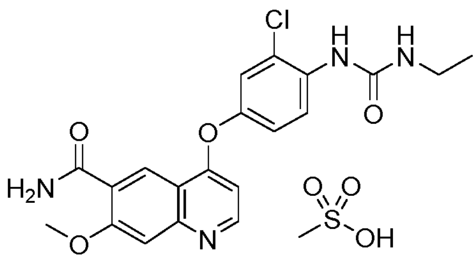

After adding methanesulfonic acid (188 μL, 2.89 mmol) at 70°C to a suspension of 4-(3-chloro-4-(ethylaminocarbonyl)aminophenoxy)-7-methoxy-6-quinolinecarboxamide (1.00 g, 2.41 mmol) in methanol (20 mL), the mixture was stirred for 15 minutes. The reaction mixture was cooled to room temperature and stirred for 16 hours, after which the precipitated solid was filtered. The obtained solid was further washed with methanol and diethyl ether and then dried under reduced pressure to obtain the title compound (1.06 g).
$^1$H-NMR Spectrum (500 MHz, DMSO-$d_6$) $\delta$(ppm): 1.09(3H, t, J=7.1 Hz), 2.34(3H, s), 3.08-3.22(2H, m), 4.09(3H, s), 6.95(1H, d, J=6.3 Hz), 7.06(1H, brt, J=4.9 Hz), 7.34(1H, dd, J=9.0, 2.7 Hz), 7.61-7.66(2H, m), 7.91(1H, brs), 7.97(1H, brs), 8.16(1H, s), 8.36(1H, d, J=9.3 Hz), 8.73(1H, s), 8.97(1H, d, J=6.3 Hz).

## Claims

**1.** A liposome composition comprising:

(A) liposomes comprising a dihydrosphingomyelin and a diacylglycerol-polyethylene glycol,
(B) a drug, and
(C) a sulfuric acid salt and/or sucrose octasulfate,

wherein

(A) encapsulates (B),
the diacylglycerol-polyethylene glycol is distearoylglycerol-polyethylene glycol, and
(B) is 4-(3-chloro-4-(ethylaminocarbonyl)aminophenoxy)-7-methoxy-6-quinolinecarboxamide represented by formula (I) or its pharmaceutically acceptable salt.

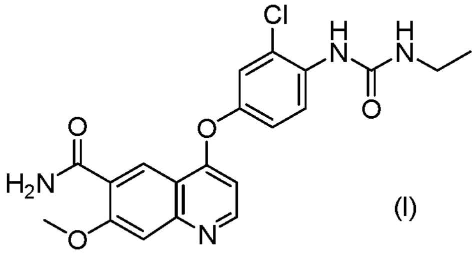

(I)

2. The liposome composition according to claim 1, wherein (C) is a sulfuric acid salt.

3. The liposome composition according to claim 1 or 2, wherein the dihydrosphingomyelin is a compound represented by formula (II-1), (II-2), (II-3) or (II-4), or their mixture.

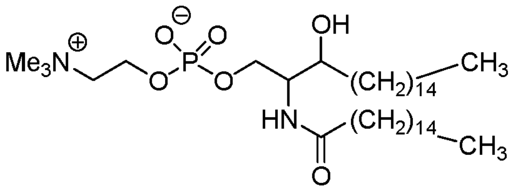 (II-1)

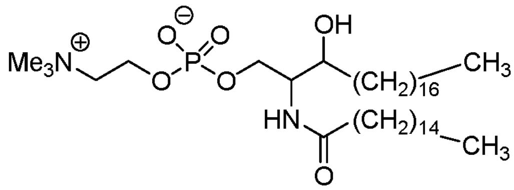 (II-2)

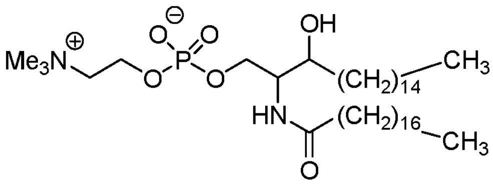 (II-3)

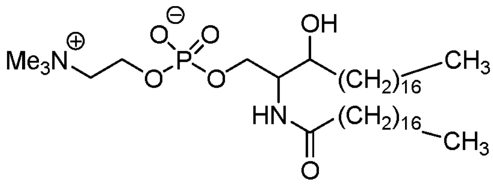 (II-4)

4. The liposome composition according to claim 1 or 2, wherein the dihydrosphingomyelin is a compound represented by formula (II-3).

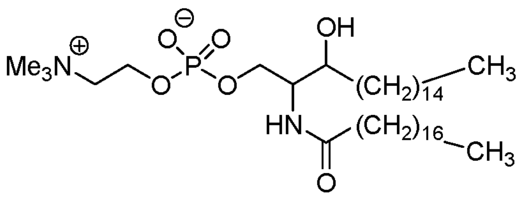 (II-3)

5. The liposome composition according to any one of claims 1 to 4, further comprising (D) an organic acid.

6. The liposome composition according to claim 5, wherein (D) is one or more selected from the group consisting of succinic acid, citric acid, maleic acid and salicylic acid.

7. The liposome composition according to any one of claims 1 to 6, wherein (A) further comprises a cholesterol.

8. The liposome composition according to any one of claims 1 to 7, further comprising (E) a basic compound.

9. The liposome composition according to any one of claims 1 to 8, wherein (C) is ammonium sulfate.

10. The liposome composition according to any one of claims 5 to 9, wherein (D) is succinic acid.

**11.** The liposome composition according to any one of claims 8 to 10, wherein (E) is sodium hydroxide or ammonia.

**12.** A pharmaceutical composition comprising a liposome composition according to any one of claims 1 to 11.

**13.** An angiogenesis inhibitor comprising a liposome composition according to any one of claims 1 to 11.

**14.** An antitumor agent comprising a liposome composition according to any one of claims 1 to 11.

**15.** A method for producing a liposome composition according to any one of claims 1 to 11, the method comprising:

a step of providing a liposome dispersion, and
a step of mixing 4-(3-chloro-4-(ethylaminocarbonyl)aminophenoxy)-7-methoxy-6-quinolinecarboxamide represented by formula (I) or its pharmaceutically acceptable salt with the liposome dispersion.

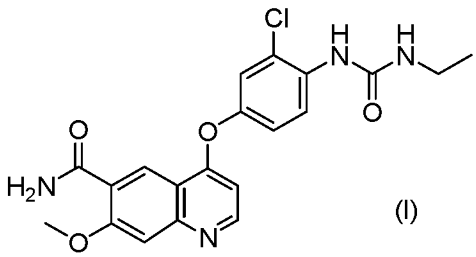

(I)

*Fig.1*

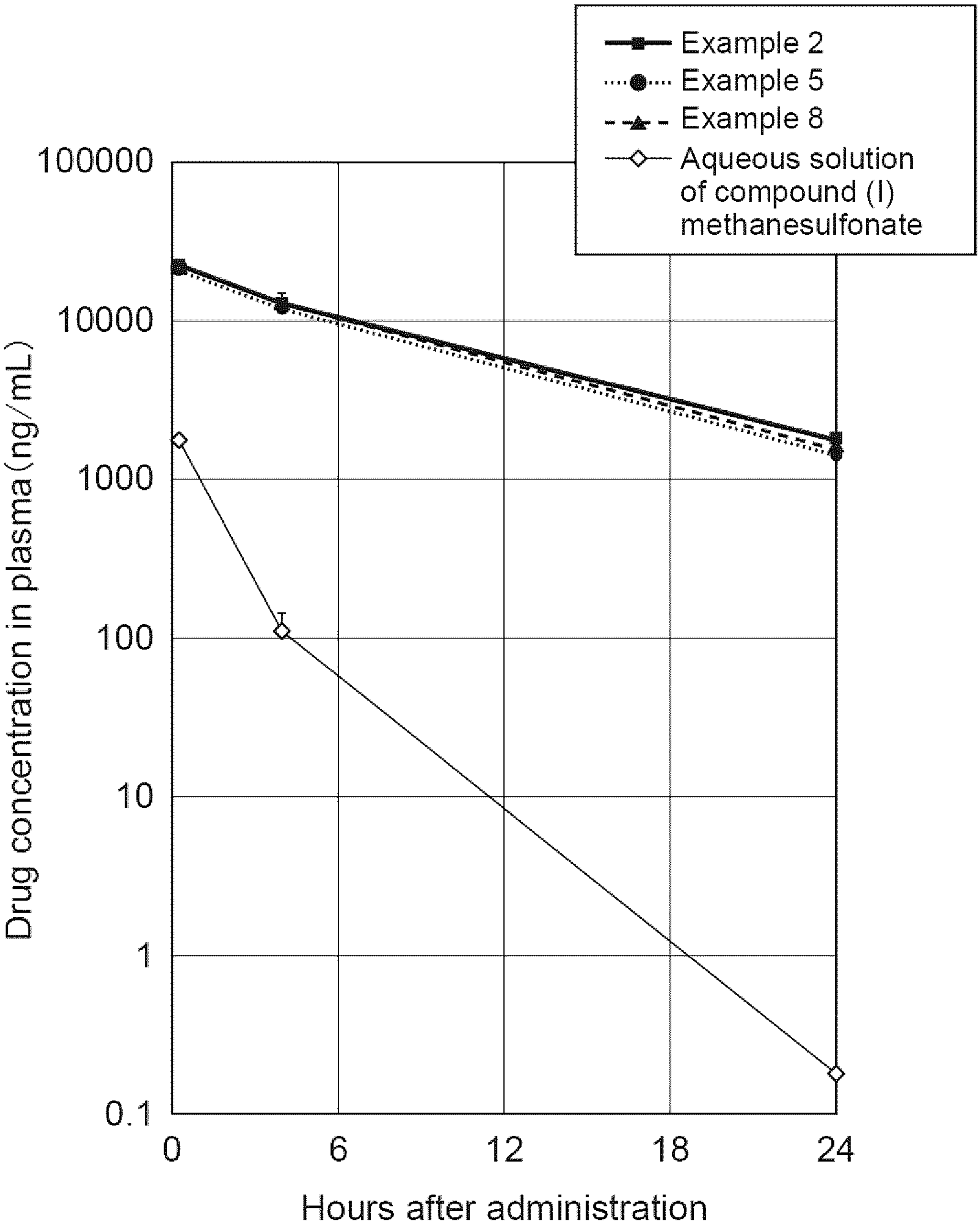
Example 2
Example 5
Example 8
Aqueous solution of compound (I) methanesulfonate
100000
10000
1000
100
10
1
0.1
Drug concentration in plasma (ng/mL)
0
6
12
18
24
Hours after administration

*Fig.2*

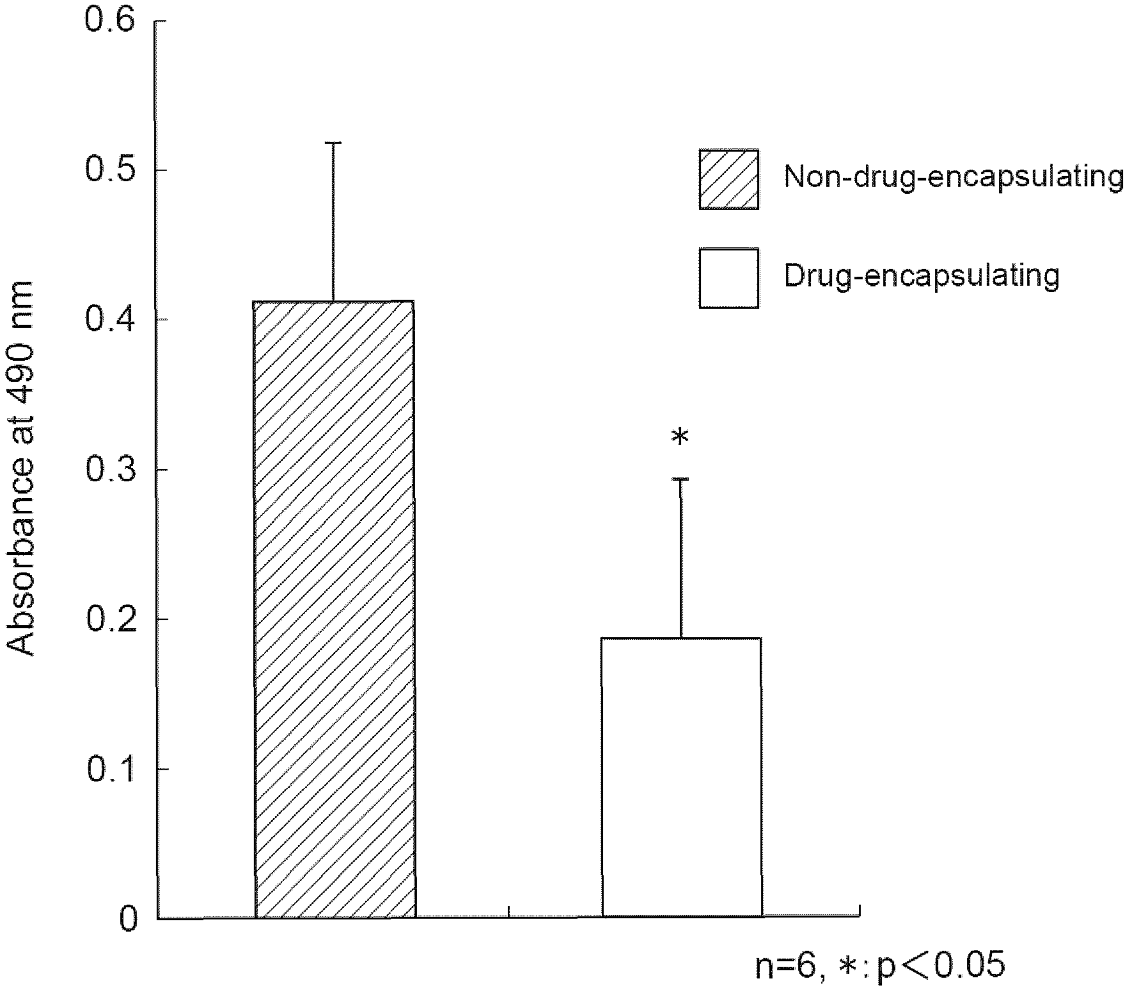
0.6
0.5
0.4
0.3
0.2
0.1
0
Absorbance at 490 nm
Non-drug-encapsulating
Drug-encapsulating
*
n=6, *: p<0.05

## INTERNATIONAL SEARCH REPORT

International application No.
**PCT/JP2023/012862**

**A. CLASSIFICATION OF SUBJECT MATTER**

***A61K 9/127***(2006.01)i; ***A61K 31/47***(2006.01)i; ***A61K 47/02***(2006.01)i; ***A61K 47/12***(2006.01)i; ***A61K 47/14***(2017.01)i; ***A61K 47/24***(2006.01)i; ***A61K 47/26***(2006.01)i; ***A61K 47/28***(2006.01)i; ***A61P 35/00***(2006.01)i; ***C07D 215/48***(2006.01)i
FI: A61K9/127; A61K47/24; A61K47/14; A61K47/02; A61K47/26; A61K31/47; A61K47/12; A61K47/28; A61P35/00; C07D215/48

According to International Patent Classification (IPC) or to both national classification and IPC

**B. FIELDS SEARCHED**

Minimum documentation searched (classification system followed by classification symbols)

A61K9/127; A61K31/47; A61K47/02; A61K47/12; A61K47/14; A61K47/24; A61K47/26; A61K47/28; A61P35/00; C07D215/48

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2012/144463 A1 (EISAI R&D MANAGEMENT CO., LTD.) 26 October 2012 (2012-10-26)<br>claims 1, 4 | 1-15 |
| A | WO 2006/030941 A1 (EISAI CO., LTD.) 23 March 2006 (2006-03-23)<br>claims 1, 21 | 1-15 |
| A | WO 2021/226368 A1 (NANOTECH PHARMA INC.) 11 November 2021 (2021-11-11)<br>claims 1, 9 | 1-15 |
| A | CN 112603890 A (SHAOXING UNIVERSITY) 06 April 2021 (2021-04-06)<br>claims | 1-15 |
| A | WO 2018/181963 A1 (FUJIFILM CORP.) 04 October 2018 (2018-10-04)<br>claims, paragraph [0041] | 1-15 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **12 June 2023** | **20 June 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (January 2015)

**INTERNATIONAL SEARCH REPORT**
**Information on patent family members**

International application No.
**PCT/JP2023/012862**

| Patent document cited in search report | Publication date (day/month/year) | Patent family member(s) | Publication date (day/month/year) |
|---|---|---|---|
| WO 2012/144463 A1 | 26 October 2012 | US 8962650 B2<br>claims 1, 3<br>EP 2700403 A1<br>CN 103402519 A | |
| WO 2006/030941 A1 | 23 March 2006 | US 2006/0135486 A1<br>claims 1, 21<br>EP 1797877 A1<br>KR 10-2007-0114774 A<br>CN 101232901 A | |
| WO 2021/226368 A1 | 11 November 2021 | CN 115605196 A<br>claims 1, 9 | |
| CN 112603890 A | 06 April 2021 | (Family: none) | |
| WO 2018/181963 A1 | 04 October 2018 | US 2020/0016079 A1<br>claims, paragraph [0086]<br>EP 3603620 A1<br>CN 110505869 A<br>KR 10-2019-0120319 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018181963 A
- US 7253286 B
- CN 112603890


### Non-patent literature cited in the description


- *CHEMICAL ABSTRACTS*, 417719-46-1
- **SHINSEIKAGAKU JIKKEN KOZA**. *Cell Culture Technology*, 197-202
- *Reference A: International Journal of Pharmaceutics*, 2012, vol. 15, 636-643